# EUROPEAN PATENT APPLICATION

(11) **EP 3 949 838 A1**
(43) Date of publication of application: **09.02.2022**
(21) Application number: 20784323.6
(22) Date of filing: 27.03.2020
(51) Int. Cl.: A61B 5/02, A61B 5/0245, A61B 5/08, A61B 5/11, A61B 5/113, A61B 5/16

(54) **SLEEP STATE DETECTION DEVICE, SLEEP STATE DETECTION METHOD, AND SLEEP STATE DETECTION PROGRAM**

(30) Priority: 03.04.2019 JP 2019071638; 03.04.2019 JP 2019071657; 03.04.2019 JP 2019071645
(71) Applicant: Teijin Limited, Osaka 530-0005 (JP)
(72) Inventor: SUZUKI, Takuji, Osaka-shi, Osaka 530-0005 (JP); ARATANI, Wataru, Osaka-shi, Osaka 530-0005 (JP)
(74) Representative: Hoffmann Eitle
(86) International application number: PCT/JP2020/014397
(87) International publication number: WO 2020/203935

(57) **Abstract**

A simple and convenient sleep condition detection apparatus with a high precision of detection of an apnea or hypopnea condition at the time of sleep, the sleep condition detection apparatus (2, 3) comprising an acquisition part (221, 321) for acquiring beat interval data of a test subject in a predetermined period including a sleep period, an event detection part (222, 322) for detecting CVHR event data from the beat interval data, an event processing part (223, 323) for performing exclusion processing for excluding from the CVHR event data the CVHR event data of a period where the beat interval becomes within a predetermined range, and an output part (224, 324) for outputting an apnea indicator signal related to the processed CVHR event data.

## Description

### FIELD

The present disclosure relates to a sleep condition detection apparatus, sleep condition detection method, and sleep condition detection program.

### BACKGROUND

Sleep apnea syndrome (SAS) is a disorder in which breathing stops or breathing becomes shallow at the time of sleep. As the method of diagnosis of the sleep apnea syndrome, there is a sleep polygraph examination measuring the brainwaves (EEG: electroencephalography), eyeball motion (EOG: electrooculography), ECG: electrocardiogram, EMG: electromyography, breathing curve, snoring, arterial blood oxygen saturation, etc. during sleep overnight. However, there are the problems that the apparatus becomes bulky, the test subject has to check in the hospital, and measurement is not easy. Further, comprehensive judgment by a physician or clinical technician is required. Therefore, there is a great need for a sleep condition detection apparatus able to simply diagnose the sleep apnea syndrome.

The fact that a condition where apnea or hypopnea occurs at the time of sleep is related to variation in the heart rate is known. If observing the changes in the heart rate at the time of sleep, compared with the variation in the heart rate at the time of normal sleep, when apnea or hypopnea occurs, a distinctive variation of heart rate where a slow pulse and rapid pulse alternately repeat, a so-called "cyclic variation of heart rate" (CVHR), occurs.

PTL 1 discloses a method of detecting the CVHR and judging a condition where apnea or hypopnea occurs. Furthermore, to improve the precision of judgment of the apnea or hypopnea condition, it discloses utilizing the changes in the activity ratios of the sympathetic and parasympathetic nerves as indicators reflecting the health conditions of the test subjects.

PTL 2 discloses a method of judgment of a sleep condition which finds activity ratios of the sympathetic and parasympathetic nerves from the heart beat intervals to diagnose REM sleep, nonREM sleep, and other sleep conditions.

PTL 3 discloses a method of judgment of a sleep condition which uses pulse interval data during sleep to judge a sleep condition of a test subject during which it excludes corresponding pulse interval data when body movement showing a degree of movement of the body of the test subject is in a predetermined range.

PTL 4 discloses a display method for visually displaying data relating to a sleep condition of a user while linking a time getting out of bed and a time getting into bed on a time display screen with an area, number, or color of graphics or a combination of the same.

### [CITATIONS LIST]

### [PATENT LITERATURE]

[PTL 1] Japanese Unexamined Patent Publication No. 2010-051387
[PTL 2] Japanese Unexamined Patent Publication No. 7-143972
[PTL 3] Japanese Unexamined Patent Publication No. 2005-279113
[PTL 4] Japanese Unexamined Patent Publication No. 2013-146463

### SUMMARY

In the detection method described in PTL 1, in the case of REM sleep etc., it is known to obtain the variation in heart rate - which is similar to the CVHR. The variation in heart rate at the time of REM sleep etc. may be mistakenly detected as stopped breathing or shallow breathing at the time of sleep.

The method of diagnosis described in PTL 2 can judge REM sleep and nonREM sleep, but judgment of an apnea or hypopnea condition at the time of sleep is not disclosed.

As the cause of body movement of a test subject, there are various factors in addition to the apnea or hypopnea of the test subject. It is difficult to judge apnea or hypopnea by just body movement disclosed in PTL 3.

PTL 4 does not disclose a method of display of an apnea sleep condition or REM sleep and nonREM sleep conditions.

The sleep condition detection apparatus is preferably a simple and convenient sleep condition detection apparatus with a high precision of detection of an apnea or hypopnea condition at the time of sleep.

The sleep condition detection apparatus according to one aspect of the embodiments comprises an acquisition part for acquiring beat interval data of a test subject in a predetermined period including a sleep period, an event detection part for detecting CVHR event data from the beat interval data, an event processing part for performing exclusion processing for excluding from the CVHR event data the CVHR event data of a period where the beat interval becomes within a predetermined range, and an output part for outputting an apnea indicator signal related to the processed CVHR event data.

Preferably, the sleep condition detection apparatus according to one aspect of the embodiments further comprises an autonomous nerve indicator output part for outputting autonomous nerve indicator data showing an autonomous nerve indicator based on the beat interval data, and the event processing part further excludes from the processed CVHR event data the CVHR event data of a period where the autonomous nerve indicator data falls below a predetermined threshold.

Preferably, the sleep condition detection apparatus according to one aspect of the embodiments further comprises an autonomous nerve indicator output part for outputting autonomous nerve indicator data from the beat interval data and a breathing diagnosis part for diagnosing an apnea or hypopnea sleep condition of the test subject based on the processed CVHR event data and the autonomous nerve indicator data, and the output part outputs an apnea indicator signal showing the results of diagnosis.

Preferably, the sleep condition detection apparatus according to one aspect of the embodiments further comprises a body movement data output part for acquiring a body movement signal of the test subject and outputting body movement data, and the breathing diagnosis part diagnoses an apnea or hypopnea sleep condition of the test subject based on the beat interval data, the autonomous nerve indicator data, and the body movement data.

Preferably, in the sleep condition detection apparatus according to one aspect of the embodiments, the breathing diagnosis part further comprises an event detection part for detecting CVHR event data from the beat interval data and an event processing part for excluding from the CVHR event data the CVHR event data of a period where the beat interval becomes within a predetermined range, excludes from the CVHR event data the CVHR event data of a period where the autonomous nerve indicator data becomes less than a predetermined autonomous nerve indicator threshold, and excludes the CVHR event data of a period where the body movement data becomes less than a predetermined body movement threshold.

Preferably, in the sleep condition detection apparatus according to one aspect of the embodiments, the breathing diagnosis part diagnoses an apnea or hypopnea sleep condition based on data of a plurality of test subjects.

The sleep condition detection method according to one aspect of the embodiments comprises acquiring beat interval data of a test subject in a predetermined period including a sleep period, detecting CVHR event data from the beat interval data, performing exclusion processing excluding from the CVHR event data the CVHR event data of a period where the beat interval becomes within a predetermined range, and outputting an apnea indicator signal relating to the processed CVHR event data.

Preferably the sleep condition detection method according to one aspect of the embodiments further comprises outputting autonomous nerve indicator data showing an autonomous nerve indicator from the beat interval data, diagnosing an apnea or hypopnea sleep condition of the test subject based on the processed CVHR event data and the autonomous nerve indicator data, and outputting an apnea indicator signal showing the results of diagnosis.

Preferably the sleep condition detection method according to one aspect of the embodiments further comprises acquiring a sleep condition indicator based on beat interval data of the test subject, acquiring an autonomous nerve indicator based on the beat interval data, acquiring CVHR events showing the occurrence of an apnea condition of the test subject based on the sleep condition indicator and the autonomous nerve indicator, acquiring an apnea indicator based on the CVHR events, displaying the sleep condition indicator, the autonomous nerve indicator, and the apnea indicator at predetermined common time intervals in a time series, and displaying the CVHR events overlapped with other indicators.

Preferably in the sleep condition detection method according to one aspect of the embodiments, the CVHR events are displayed overlapped with the sleep condition indicator, the autonomous nerve indicator, and the apnea indicator so as to cover over the display of the indicators.

Preferably in the sleep condition detection method according to one aspect of the embodiments, the CVHR events are displayed overlapped with the sleep condition indicator, the autonomous nerve indicator, and the apnea indicator so as to enable the display of the indicators to be viewed.

The sleep condition detection program according to one aspect of the embodiments is a sleep condition detection program for running a computer, which program makes a computer run the above-mentioned sleep condition detection method.

According to the present embodiments, the sleep condition detection apparatus, sleep condition detection method, and sleep condition detection program can simply and conveniently improve the precision of detection of an apnea or hypopnea condition at the time of sleep.

The object and effects of the present invention will be able to be understood and obtained by using the constituent elements pointed out in particular in the claims and combinations of the same. Both of the above-mentioned general explanation and the later explained detailed description are illustrative and explanatory and do not limit the present invention described in the claims.

### BRIEF DESCRIPTION OF DRAWINGS

FIGS. 1A and 1B are an electrocardiogram showing beats of the heart and a view explaining a heart beat interval as the time interval of beats of the heart. FIG. 1A shows an electrocardiogram waveform, while FIG. 1B show a graph of the R-R interval (RRI).
FIG. 2 is a view for explaining a method of diagnosing a CVHR from the conventional variation in a pulse wave.
FIG. 3 is a view showing one example of a conventional algorithm for diagnosing CVHR flags from a pulse wave.
FIG. 4 is a view showing a graph plotting the relationship of a smoothed pulse wave and CVHR events.
FIG. 5 is a view showing one example of a summary of processing of a first embodiment of a sleep condition detection apparatus.
FIG. 6 is a view showing one example of the configuration of the first embodiment of a sleep condition detection apparatus.
FIG. 7 is a view showing one example of a flow chart of sleep condition detection processing performed in the first embodiment.
FIG. 8 is a view showing one example of an autonomous nerve indicator (LF/HF).
FIG. 9 is a view showing one example of a summary of processing of a second embodiment of a sleep condition detection apparatus.
FIG. 10 is a view showing one example of the configuration of the second embodiment of a sleep condition detection apparatus.
FIG. 11 is a view showing one example of a flow chart of sleep condition detection processing performed in the second embodiment.
FIG. 12 is a view showing the effects of detection of the CVHR according to the present disclosure.
FIG. 13 is a view showing one example of a summary of processing of a third embodiment of a sleep condition detection apparatus.
FIG. 14 is a view showing one example of the configuration of the third embodiment of a sleep condition detection apparatus.
FIG. 15 is a view showing one example of a flow chart of sleep condition detection processing performed in the third embodiment.
FIGS. 16A and 16B are perspective views of the shape showing one example of a fourth embodiment of a sleep condition detection apparatus. FIG. 16A is a perspective view of the shape from the front side, while FIG. 16B is a perspective view of the shape from the back side.
FIG. 17 is a view showing one example of the configuration of a main body part of the fourth embodiment of a sleep condition detection apparatus.
FIG. 18 is a view showing one example of a summary of processing of a sleep condition detection apparatus used in a sleep condition display.
FIG. 19 is a view showing one example of the configuration of a sleep condition detection apparatus used in a sleep condition display.
FIG. 20 is a view showing one example of a sleep display screen.
FIG. 21 is a view showing one example of the configuration of a sleep condition display apparatus.
FIG. 22 is a view showing one example of a flow chart of sleep condition display processing.

### DESCRIPTION OF EMBODIMENTS

Below, a sleep condition detection apparatus, sleep condition detection method, and sleep condition detection program according to aspects of the present disclosure will be explained while referring to the drawings. However, the technical scope of the present disclosure is not limited to these embodiments. It should be noted that it extends to the inventions described in the claims and their equivalents. Note that, in the following explanation and drawings, constituent elements having the same functions and configurations will be assigned the same notations and thereby overlapping explanations will be omitted.

### Beats of Heart and Heart Beat Interval

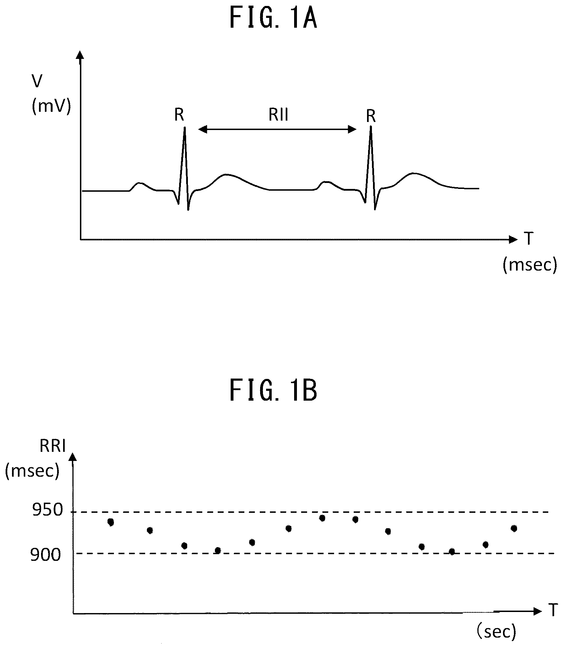

FIGS. 1A and 1B are an electrocardiogram showing beats of the heart and a view explaining the heart beat interval (R-R interval: RRI) as the time interval of beats of the heart. FIG. 1A shows an electrocardiogram waveform, while FIG. 1B shows a graph of the heart beat interval (RRI).

It is well known that when a person lies down or otherwise rests, the beats of his or her heart become "slower" while when exercising or when stressed, the beats of his or her heart become "faster". The phenomenon of the beats of the heart becoming "fast" or "slow" can, for example, be expressed by the "heart beat interval" of the time between one beat and the next beat. If the beats of the heart are fast, the heart beat interval time becomes smaller, while conversely if the beats of the heart are slow, the heart beat interval time becomes larger.

FIG. 1A is a view of an electrocardiogram showing an electrical signal generated when the heart is beating. The sharpest peak R of the electrocardiogram is an electrical signal generated when the part of the heart called the ventricle rapidly contracts and pumps out blood from the heart. The time interval between two R's will be called the "R-R Interval" (RRI).

FIG. 1B is a graph plotting the R-R interval (RRI). The R-R interval (RRI), it is known, is not always constant, but fluctuates. By exercising, the heart rate rises and the R-R interval (RRI) changes. Not only that, variation of the R-R interval (RRI) is observed even if staying quietly still or if sleeping. In the top view, the R-R interval (RRI) varies from 950 msec to 900 msec. Such variation of the heart beat interval is called "heart rate variability".

When a test subject suffers from an apnea or hypopnea sleep condition, it is known that a characteristic heart rate variability of an alternately repeating slow pulse and fast pulse, a so-called "CVHR", arises. However, there are various factors in the CVHR. It is necessary to simply and precisely diagnose when a test subject suffers from an apnea or hypopnea sleep condition. Further, in the present embodiment, the R-R interval (RRI) and a P-P interval (PPI) will together be referred to as the "beat interval". The P-P interval (PPI), for example, means the time interval between the sharpest peaks of a pulse wave. An electrocardiogram obtains the changes in potential occurring along with activity of the muscle of the heart, while the pulse wave measures the variation in the flow of blood pumped out by the heart through the arteries to the extremities. The RRI and the PPI are information respectively acquired from the same, but it is known that these show substantially similar values at the time of sleep and other relatively resting times. In the following embodiments, the explanation will be given based on a pulse wave.

### Conventional Method of Diagnosis of CVHR From Variation of Pulse Wave

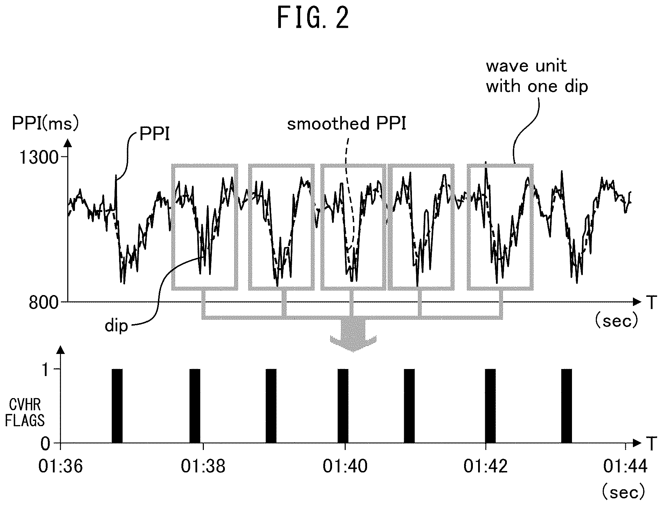

FIG. 2 is a view for explaining the conventional method of diagnosis of the CVHR from variation of a pulse wave.

The top graph of FIG. 2 is a graph plotting a pulse wave value on the ordinate and a sleep time on the abscissa. The solid line shows the actual pulse wave, while the broken line is, for example, the pulse wave smoothed by the moving average. A drop in the smoothed pulse wave will be called a "dip". The bottom graph of FIG. 2 is a graph plotting the CVHR flags diagnosed from the smoothed pulse wave with the sleep time as the abscissa. "CVHR flags" are flags showing the test subject suffers from an apnea condition.

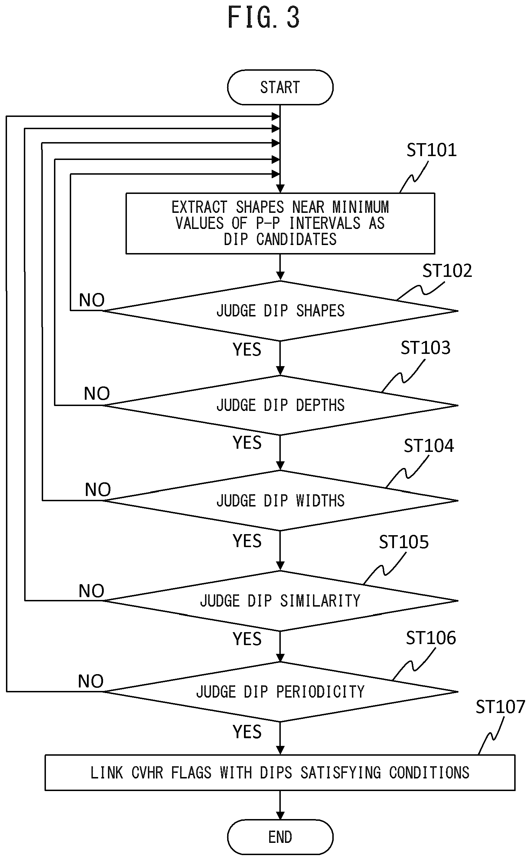

FIG. 3 is a view showing one example of a conventional algorithm for diagnosing CVHR flags from a pulse wave. First, it extracts shapes near the minimum values of the smoothed pulse wave as dip candidates (ST101). The judgment of the extracted dip shapes, for example, judges if the dip shapes are similar to a predetermined parabolic shape (ST102). Furthermore, it calculates the depths of the dips, for example, the depths of the individual dips, as the difference between the values at the center times of the minimal values and the average value of preceding and succeeding maximum values of the moving average, to judge the threshold (ST103). Furthermore, it judges the widths of the dips, for example, judges if they have within a predetermined range of dip width with respect to the adjoining dips (ST104).

Furthermore, it judges the similarity of the dips (ST105). For example, it identifies dips having within a predetermined range of dip width and within a predetermined range of dip height with respect to the adjoining dips and having within a predetermined range of ratio of dip width and dip height with respect to the adjoining dips as dips having similarity. Furthermore, it judges the periodicity of the dips (ST106). For example, if, for all of the two adjoining dips among four consecutive dips, the time difference of the two adjoining dips is within a predetermined range of time and the variation in magnitude of the time difference is within a predetermined range, the four dips are deemed to have periodicity. The dips satisfying all of the above conditions are acquired as CVHR flags (ST107). CVHR flags show that the test subject suffers from an apnea condition at the times corresponding to the CVHR flags and that CVHR events have occurred.

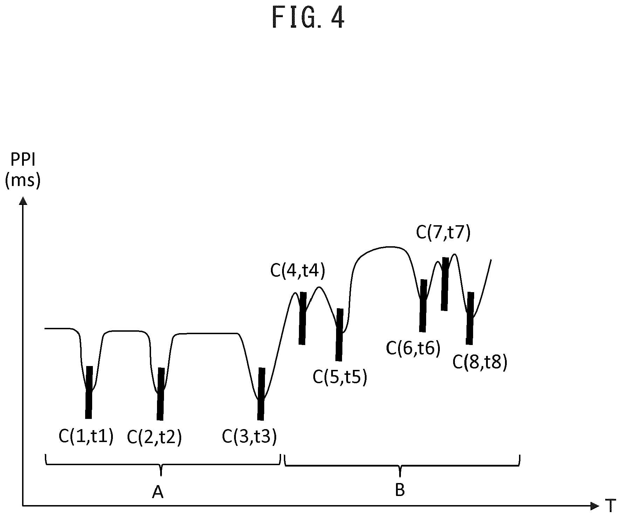

FIG. 4 is a view showing a graph plotting the relationship of a smoothed pulse wave and CVHR events.

The smoothed pulse wave of the test subject, it will be understood, is stable at the left side part A, but greatly varies at the right side part B. Using a conventional algorithm for diagnosing the CVHR from variation of the pulse wave, at the left side part A, the CVHR flags c(1, t1), c(2,t2), c(3, t3) are acquired. Here, c(n,tn) is the n-th CVHR flag and shows detection at the time tn (n: natural number). Furthermore, at the right side part B, the CVHR flags c(4, t4), c(5,t5), c(6, t6), c(7,t7), c(8, t8) are acquired.

At the right side part B where the smoothed pulse wave greatly varies, the test subject does not suffer from an apnea condition, but for example is in a REM sleep or another sleep condition. This fact was learned by the inventors by comparing an actual apnea condition with the detected CVHR flags. The present invention excludes from the CVHR flags acquired by the conventional algorithm acquiring the CVHR from the variation of a pulse wave the CVHR flags where the test subject does not suffer from an apnea condition so as to improve the precision of detection of an apnea sleep condition of the test subject.

### Summary of Processing of First Embodiment of Sleep Condition Detection Apparatus

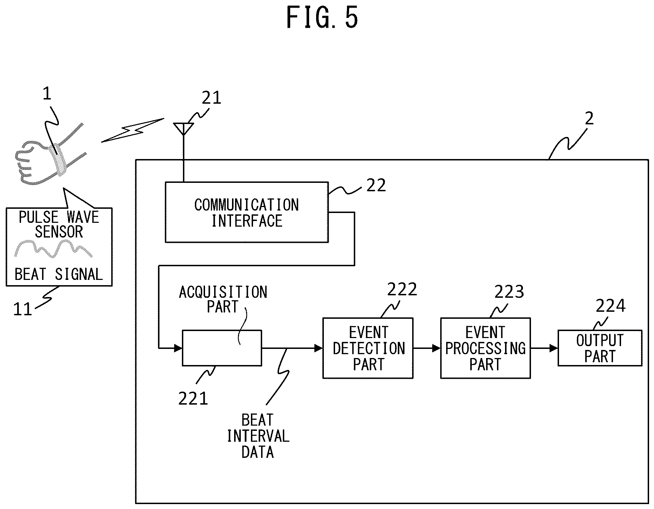

FIG. 5 is a view showing one example of the summary of processing of the first embodiment of a sleep condition detection apparatus.

At the wrist of the test subject, a wristband 1 having an optical type pulse wave sensor 11 detecting the beating condition of the heart of the test subject, for example, that is, the pulse, is attached. The optical type pulse wave sensor 11, for example, has an optical type pulse wave sensor IC mounting an LED driver and green color detection photodiode. The intensity of reflected light at the time of firing the light from the LED toward the inside of the body is measured by the photodiode to obtain a pulse wave signal. The pulse wave sensor may be not only an optical type, but also, for example, a pulse pressure detection type pulse wave sensor.

In the present embodiment, the state is shown with the wristband 1 attached to the wrist of the test subject, but it may also be attached to another portion of the test subject.

The beat signal detected by the optical type pulse wave sensor 11 of the wristband 1 is sent by wireless communication, for example WiFi or Bluetooth^{®}, to the sleep condition detection apparatus 2. The beat signal need not be sent in real time. For example, it may be sent to the sleep condition detection apparatus 2 all together after the test subject wakes up from sleep. Alternatively, there is no need to directly wirelessly send the beat signal from the wristband 1 to the sleep condition detection apparatus 2. For example, it may be sent to the sleep condition detection apparatus 2 through a smart phone or access point.

The beat signal sent from the wristband 1 is received by a communication interface 22 through an antenna 21 of the sleep condition detection apparatus 2. An acquisition part 221 acquires the beat interval data of the test subject from the beat signal in a predetermined period including a sleep period.

An event detection part 222 smooths the beat interval data. Furthermore, the event detection part 222 uses the conventional algorithm for diagnosing CVHR flags from a pulse wave shown in FIG. 3 to detect CVHR events. The detected CVHR event data is input in an event processing part 223.

The event processing part 223 excludes from the detected CVHR event data the CVHR event data corresponding to a period where the beat interval is within a predetermined range. In the CVHR accompanying apnea, the sympathetic nerves become dominant and the average beat interval tends to become short, but for example in the case of REM sleep, the variation of heart rate becomes similar to the CVHR, but the average beat interval tends to be longer than the case of apnea, so the predetermined range is made the vicinity of the average beat interval at the time of REM sleep. By excluding this range, only the CVHR event data showing apnea is selected.

Based on the results of detection by the event processing part 223, an output part 224 outputs the CVHR event data as an apnea indicator signal. Instead of the CVHR event data or together with the CVHR event data, it is also possible to output an indicator of breathing per unit time of detection, for example, a value corresponding to the apnea hypopnea index (AHI) of the combined number of times of stopped breathing and shallow breathing per hour.

The apnea indicator signal is input to a not shown display part built into the sleep condition detection apparatus 2. On the display screen, for example, a value corresponding to the apnea hypopnea index is displayed. Alternatively, the apnea indicator signal may be written in a not shown hard disk built into the sleep condition detection apparatus 2 or a detachable memory card. Furthermore, a value corresponding to the apnea hypopnea index may be output to a printer, information terminal apparatus, or server apparatus connected to the sleep condition detection apparatus 2. Alternatively, it may be sent through the network to, for example, a server apparatus having a test subject data base.

Further, the optical type pulse wave sensor 11 may also send a beat interval signal instead of a pulse wave signal or may also send a pulse wave signal and beat interval signal.

According to the sleep condition detection apparatus 2 of the present embodiment, it is possible to simply and conveniently improve the precision of detection of an apnea or hypopnea condition at the time of sleep.

### Example of Constitution of First Embodiment of Sleep Condition Detection Apparatus

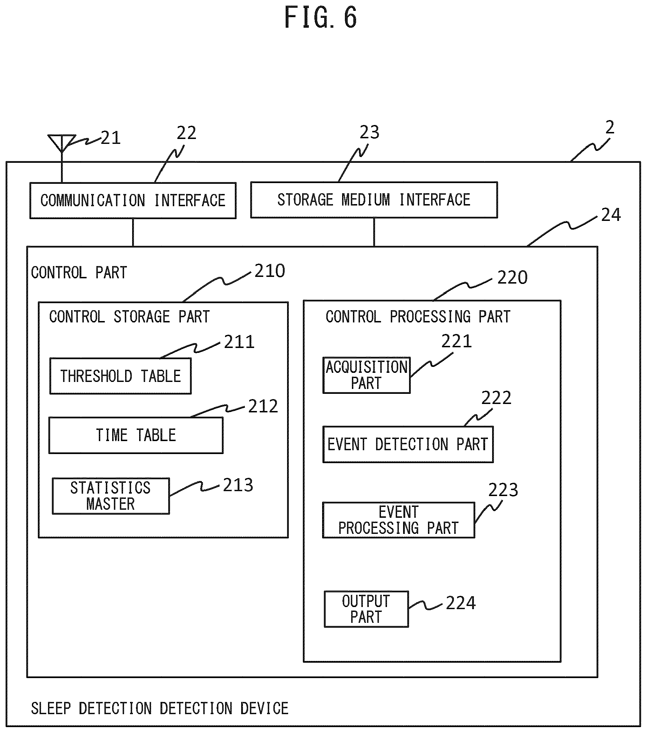

FIG. 6 is a view showing one example of the configuration of a first embodiment of a sleep condition detection apparatus.

The sleep condition detection apparatus 2 has a communication interface 22 communicating with the outside through an antenna 21 and a control part 24 connected with the communication interface 22. Furthermore, it may have a storage medium interface 23 connected to an outside storage medium. For example, using the storage medium interface 23, the sleep condition detection apparatus 2 can read detection data stored in the storage medium of the wristband 1.

The control part 24 has a control storage part 210 and a control processing part 220. The control storage part 210 is comprised of one or more semiconductor memories. For example, it has at least one of a RAM, flash memory, EPROM, EEPROM, or other nonvolatile memory. The control storage part 210 stores a driver program, operating system program, application programs, data, etc. used for processing by the control processing part 220.

For example, the control storage part 210 stores as a driver program a device driver program for controlling the communication interface 22. The computer program, for example, may be installed from a CD-ROM, DVD-ROM, or other computer readable portable storage medium using a known setup program etc. in the control storage part 210. Further, it may be installed downloaded from a program server etc.

Furthermore, the control storage part 210 may temporarily store temporary data relating to predetermined processing. The control storage part 210 stores a threshold table 211 of a dip judgment threshold, beat interval threshold, beat interval range threshold, beat interval average threshold, etc., a time table 212 of a diagnosis unit time, detection unit time, etc., statistics master 213 for statistical processing, etc.

The threshold table 211 of the beat interval threshold, beat interval range threshold, beat interval average range threshold, etc. and the time table 212 of the diagnosis unit time, detection unit time, calculation time, etc. may also be made unique set values. Alternatively, it is also possible to obtain statistics on the apnea or hypopnea sleep conditions of a plurality of test subjects and statistically set the threshold table 211 and time table 212 based on the averages, dispersions, etc. of the statistical values. Furthermore, it is also possible to store bio information of test subjects, for example, settings corresponding to the ages, genders, and body weights, in the statistics master 213 and utilize them for detection of the apnea or hypopnea sleep condition of the test subject.

The control processing part 220 has one or more processors and their peripheral circuits. The control processing part 220 comprehensively controls the overall operation of the sleep condition detection apparatus 2 and is, for example, an MCU (micro controller unit).

The control processing part 220 performs processing based on the programs stored in the control storage part 210 (operating system program, driver program, application programs, etc.) Further, the control processing part 220 may also run a plurality of programs (application programs etc.) in parallel. The control processing part 220 has an acquisition part 221, event detection part 222, event processing part 223, output part 224, etc.

These parts of the control processing part 220 may also be mounted at the control part 24 as independent integrated circuits, circuit modules, microprocessors, or firmware.

### Flow of Sleep Condition Detection Processing of First Embodiment

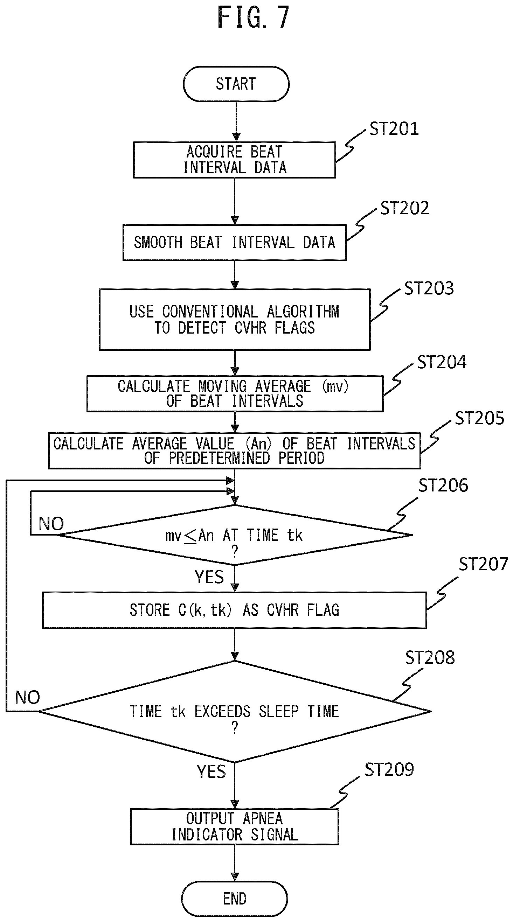

FIG. 7 is a view showing one example of a flow chart of sleep condition detection processing performed in the first embodiment.

The acquisition part 221 uses a beat signal received from the wristband 1 of the test subject to acquire beat interval data showing the beat interval (ST201).

The event detection part 222 smooths the beat interval data (ST202). Furthermore, the event detection part 222 uses the conventional algorithm for detecting CVHR flags from a pulse wave shown in FIG. 3 to detect CVHR events (ST203). The event processing part 223 calculates the moving average (mv) of the beat interval data (ST204). The event processing part 223 calculates the average value (An) in a predetermined period, for example, every hour or every sleep period (ST205).

The event processing part 223 judges if the moving average (mv) is the average value (An) or less at a time tk where a CVHR flag is detected (ST206). When the moving average (mv) is the average value (An) or less (ST206: YES), the event processing part 223 stores the CVHR flag c(k, tk) of the time tk as an CVHR flag (ST207). When the moving average (mv) is more than the average value (An) (ST206: NO), the event processing part 223 returns to ST206 where it compares the moving average (mv) and average value (An) of the next CVHR flag. Note that, here, the moving average (mv) is the average value (An) or less, but the invention is not limited to this. It may also be a constant multiple (for example 1.1) of the average value (An) etc. Further, An was made the average value in one sleep period, but it may also be made the average in an ultradian rhythm of sleep, for example, may be made the average of each 1.5 hours.

The event processing part 223 judges if the time tk has exceeded the sleep time (ST208). When the time tk has not exceeded the sleep time (ST208: NO), the routine returns to ST206. When the time tk has exceeded the sleep time (ST208: YES), the output part 224 outputs an apnea indicator signal based on the stored CVHR flags (ST209). The processing flow then ends.

### Summary of Processing of Second Embodiment of Sleep Condition Detection Apparatus

In the first embodiment, the sleep condition of the test subject was detected based on the beat interval data from the test subject during sleep. In the second embodiment, furthermore autonomous nerve indicator data is output from the beat interval data and the sleep condition of the test subject is detected based on the beat interval data and the autonomous nerve indicator data.

### Example of Autonomous Nerve Indicator

It is known that when the test subject suffers from an apnea sleep condition, the sympathetic nerves of the test subject become active. It is known that the degree of activity of the sympathetic nerves can be grasped by for example analyzing the variation of heart rate.

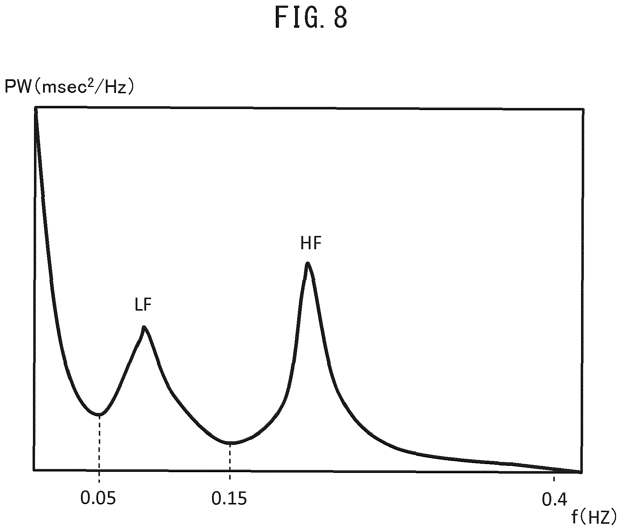

FIG. 8 is a view showing one example of an autonomous nerve indicator (LF/HF). One example of the results of analysis of the frequency of the variation of heart rate is shown. For example, it is a graph showing the relationship of the frequency "f' (abscissa) obtained by analysis of the R-R index (RRI) over a predetermined time, for example, 5 minutes, and the power PW (ordinate). The peak value of the low frequency zone (for example 0.05Hz to 0.15Hz) is designated as LF while the peak value of the high frequency zone (for example 0.15Hz to 0.4Hz) is designated as HF.

LF shows the degree of activity of mainly the sympathetic nerve functions while HF shows the degree of activity of the parasympathetic nerve functions. However, LF involves not only the sympathetic nerves, but also the parasympathetic nerves, so the ratio of LF and HF (LF/HF) is often used as an indicator of the degree of activity of the sympathetic nerves. In the present disclosure, LF/HF is used as an indicator of the degree of activity of the sympathetic nerves. Alternatively, the ratio of not the peak values, but the power spectrum density of the low frequency zone and high frequency zone may be made the autonomous nerve indicator (LF/HF). The autonomous nerve indicator (LF/HF) showing the degree of activity of the sympathetic nerves can be utilized to improve the precision of diagnosis when the test subject suffers from an apnea sleep condition.

### Summary of Processing of Second Embodiment of Sleep Condition Detection Apparatus

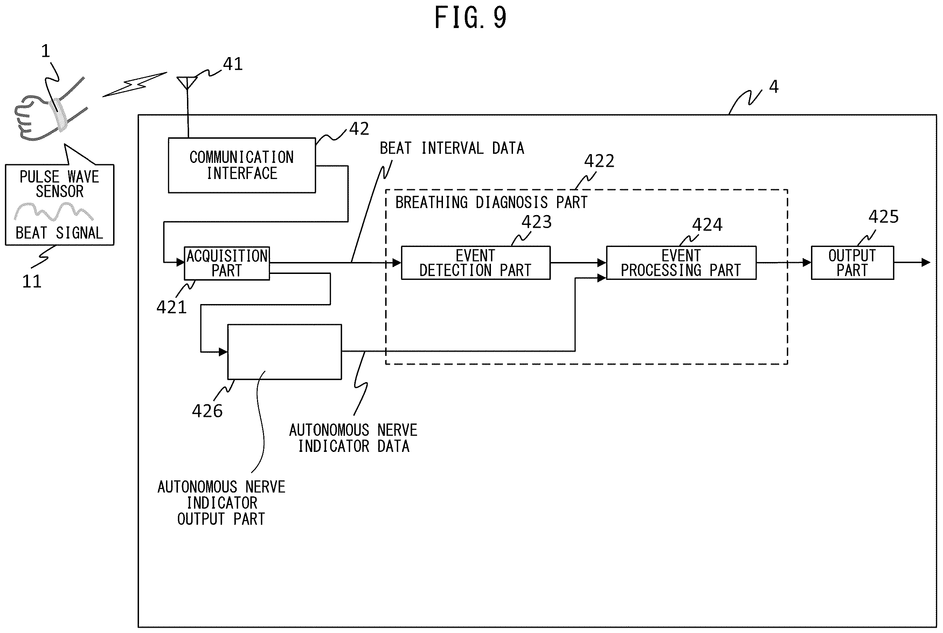

FIG. 9 is a view showing one example of a summary of processing of a second embodiment of a sleep condition detection apparatus.

The beat signal detected by the pulse wave sensor of the wristband 1 is sent by wireless communication, for example WiFi or Bluetooth^{®}. to a sleep condition detection apparatus 4.

The beat signal sent from the wristband 1 is received by a communication interface 42 through an antenna 41 of the sleep condition detection apparatus 4.

An acquisition part 421 acquires beat interval data showing the interval of beats, for example, the adjoining peak values of the pulse wave, from the beat signal showing the beating condition of the heart of the test subject. The beat interval data is input to a breathing diagnosis part 422. Alternatively, the beat interval data is output to an autonomous nerve indicator output part 426.

An event detection part 423 of the breathing diagnosis part 422 smooths the beat interval data. Furthermore, the event detection part 423 uses the conventional algorithm for detecting CVHR flags from a pulse wave shown in FIG. 3 to detect CVHR events. The detected CVHR event data is input to an event processing part 424.

An autonomous nerve indicator output part 426 outputs autonomous nerve indicator (LF/HF) data based on the beat interval data. The autonomous nerve indicator data output from the autonomous nerve indicator output part 426 is input to the event processing part 424.

The event processing part 424 excludes from the detected CVHR event data the CVHR event data corresponding to the period where the beat interval is within a predetermined range.

The event processing part 424 further filters the processed CVHR event data showing apnea based on the autonomous nerve indicator data. The filtering, for example, judges CVHR event data when the autonomous nerve indicator is a predetermined autonomous nerve indicator threshold or more as CVHR event data showing apnea. When the autonomous nerve indicator (LF/HF) is high, it is diagnosed that the possibility of the test subject suffering from an apnea or hypopnea sleep condition is high.

Based on the results of detection by the event processing part 424, an output part 425 outputs CVHR event data as an apnea indicator. Instead of the CVHR event data or together with the CVHR event data, it is also possible to output an indicator of breathing per unit time of detection, for example, a value corresponding to the apnea hypopnea index (AHI) of the combined number of times of stopped breathing and shallow breathing per hour.

The apnea indicator is input to a not shown display part built into the sleep condition detection apparatus 4. On the display screen, for example, a value corresponding to the apnea hypopnea index is displayed. Alternatively, the apnea indicator may be written in a not shown hard disk built into the sleep condition detection apparatus 4 or a detachable memory card. Furthermore, a value corresponding to the apnea hypopnea index may be output to a printer, information terminal apparatus, or server apparatus connected to the sleep condition detection apparatus 4. Alternatively, it may be sent through the network to, for example, a server apparatus having a test subject data base.

According to the sleep condition detection apparatus 4 of the present embodiment, it is possible to simply and conveniently improve the precision of detection of an apnea or hypopnea condition at the time of sleep.

### Example of Constitution of Second Embodiment of Sleep Condition Detection Apparatus

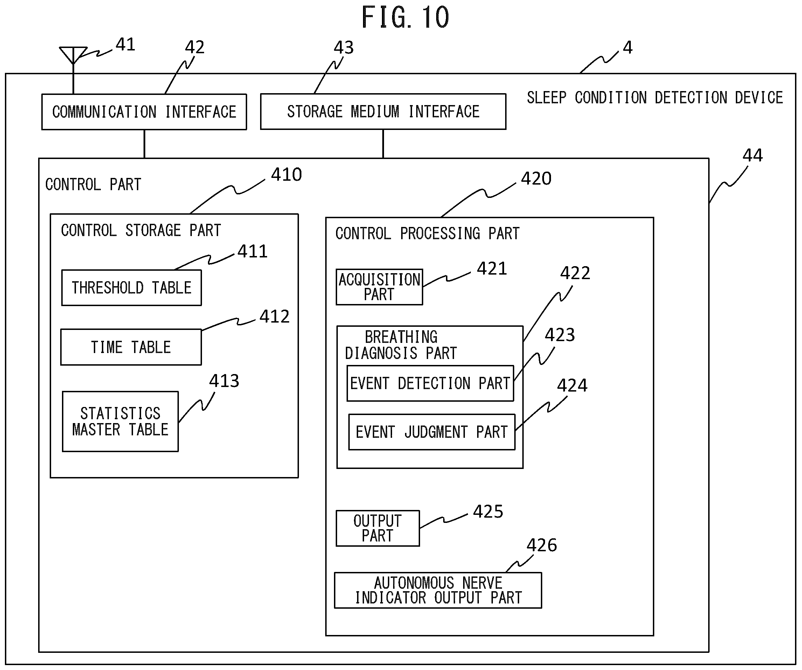

FIG. 10 is a view showing one example of the configuration of the second embodiment of a sleep condition detection apparatus.

The sleep condition detection apparatus 4 has a communication interface 42 communicating with the outside through an antenna 41 and a control part 44 for connection with the communication interface 42. Furthermore, it may have a storage medium interface 43 for connection with an outside storage medium.

The control part 44 has a control storage part 410 and a control processing part 420. The control storage part 410 is comprised of one or more semiconductor memories.

The control storage part 410 may also temporarily store temporary data relating to predetermined processing. The control storage part 410 stores a threshold table 411 of the beat threshold, beat interval average range, autonomous nerve indicator threshold, body movement threshold, etc., a time table 412 of the diagnosis time, judgment time, autonomous nerve indicator calculation time, etc., statistics master 413 for statistical processing, etc.

The control processing part 420 has an acquisition part 421, breathing diagnosis part 422, event detection part 423, event processing part 424, output part 425, autonomous nerve indicator output part 426, etc.

### Flow of Sleep Condition Detection Processing of Second Embodiment

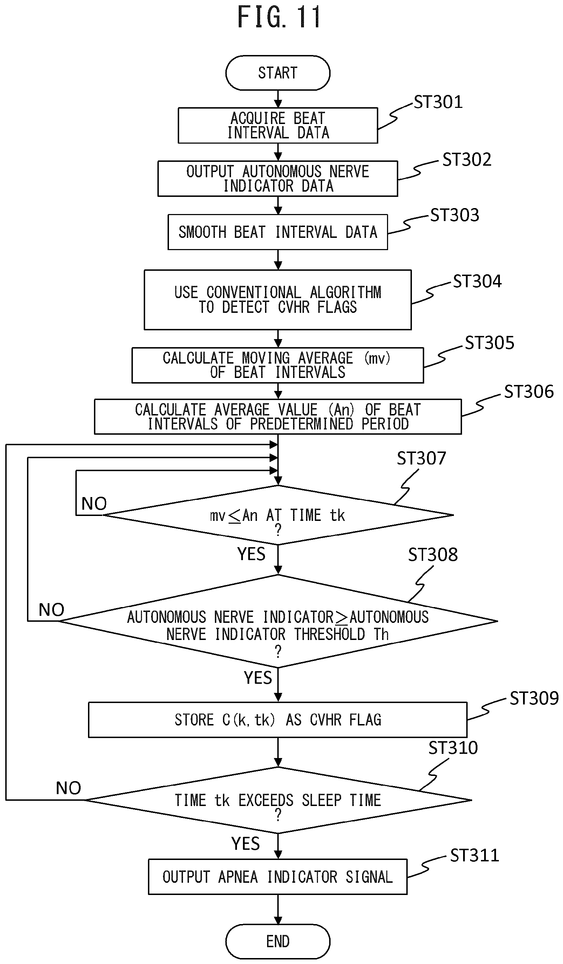

FIG. 11 is a view showing one example of a flow chart of sleep condition detection processing performed in the second embodiment.

The flow of sleep condition detection processing of the present example is processing when one sleep time's worth, for example, seven hours' worth, of the beat signal of the test subject has already been sent from the wristband 1 of the test subject to the sleep condition detection apparatus 4 and stored in the control storage part 210. The sleep condition detection processing can be performed all at once. Alternatively, for example, it is possible to acquire the beat signal in one hour units and perform diagnosis processing every hour.

The acquisition part 421 uses the beat signal received from the wristband 1 of the test subject to acquire beat interval data showing the beat interval (ST301).

The autonomous nerve indicator output part 426 outputs the autonomous nerve indicator data based on the beat interval data (ST302).

The event detection part 423 smooths the beat interval data (ST303). Furthermore, the event detection part 423 uses the conventional algorithm for detecting CVHR flags from a pulse wave shown in FIG. 3 to detect CVHR events (ST304). The event processing part 424 calculates the moving average (mv) of the beat interval data (ST305). The event processing part 424 calculates the average value (An) of the beat interval data in a predetermined period, for example, every hour or every sleep period (ST306).

The event processing part 424 judges if the moving average (mv) of the beat interval data at the time tk when a CVHR flag is detected is an average value (An) or less (ST307). When the moving average (mv) is the average value (An) or less (ST307: YES), the event processing part 224 judges if the autonomous nerve indicator at the time tk is a predetermined autonomous nerve indicator threshold Th or more (ST308). When the moving average (mv) is more than the average value (An) (ST307: NO), the event processing part 424 returns to ST307 where it compares the moving average (mv) and the average value (An) of the CVHR flags at the next time. Note that, here, the time was made when the moving average (mv) is the average value (An) or less, but the invention is not limited to this. It may also be a constant multiple (for example 1.1) of the average value (An) etc. Further, An was made the average value at one sleep period, but this may also be made the average in an ultradian rhythm of sleep, for example, may be made the average of each 1.5 hours.

When the autonomous nerve indicator at the time tk is a predetermined autonomous nerve indicator threshold Th or more (ST308: YES), the event processing part 424 stores the CVHR flag c(k, tk) of the time tk as a CVHR flag (ST309). When the autonomous nerve indicator at the time tk is less than a predetermined autonomous nerve indicator threshold Th (ST308: NO), the event processing part 424 returns to ST307 where it compares the moving average (mv) and the average value (An) of the CVHR data at the next time.

The event processing part 424 judges if the time tk exceeds the sleep time (ST310). When the time tk does not exceed the sleep time (ST310: NO), the routine returns to ST307. When the time tk has exceeded the sleep time (ST310: YES), the output part 425 outputs an apnea indicator based on the stored CVHR flags (ST311). The processing flow then ends.

### Effect of Detection of CVHR by Present Disclosure

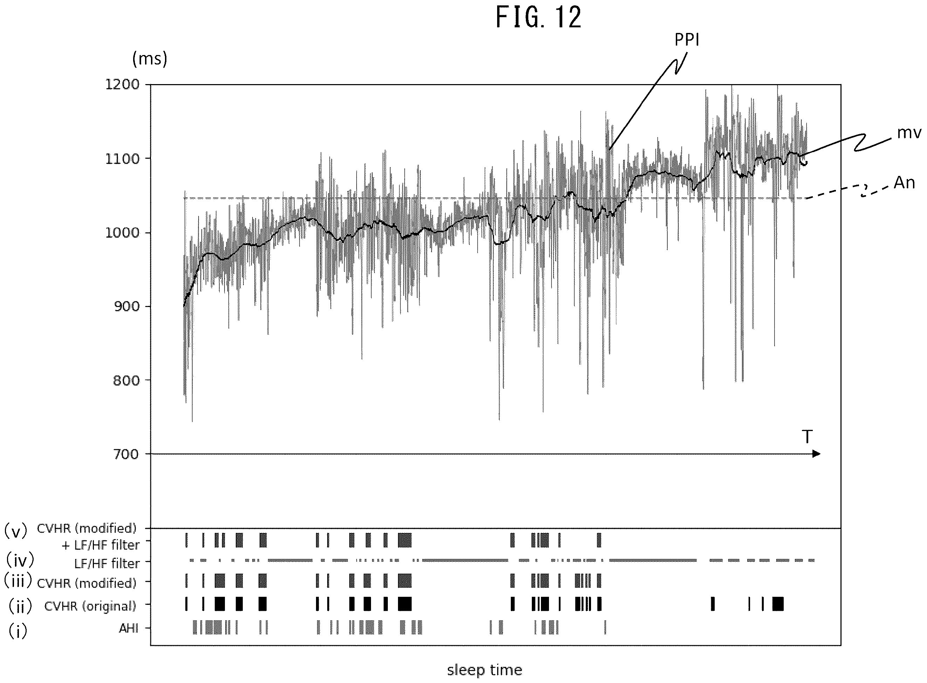

FIG. 12 is a view showing an effect of the CVHR according to the present disclosure.

At the top part of FIG. 12, the P-P interval (PPI) of one sleep period of the test subject, for example, when the sleep time is seven hours, is shown. The moving average (mv) is shown by a solid line, while the average value (An) is shown by a broken line.

The (i) AHI of the lowest level shows an actual apnea or hypopnea condition measured by a sleep polygraph apparatus in a band form. The (ii) CVHR (original) of the second level from the bottom shows the condition when detecting the occurrence of CVHR events using the conventional algorithm in a band form. If comparing (i) and (ii), it is learned that (ii) actually includes many conditions not an apnea or hypopnea condition.

The (iii) CVHR (modified) of the third level from the bottom shows the effect of detection of the sleep condition of the test subject by the first embodiment of the present application. The CVHR events when excluding cases where the moving average (mv) is longer than the average value (An) of the P-P interval (PPI) from (i) is shown in a band form. It is learned that the diagnosis is improved by the CVHR event data of a period where the beat interval is within a predetermined range being excluded from the CVHR event data detected using the conventional algorithm.

In the (iv) LF/HF filter of the fourth level from the bottom, the parts forming spaces in the horizontal bold line are parts corresponding to the parts of the autonomous nerve indicator where the autonomous nerve indicator data becomes a predetermined autonomous nerve indicator threshold or more.

The (v) CVHR (modified)+LF/HF filter of the fifth level from the bottom shows the effect of detection of the sleep condition of the test subject by the second embodiment of the present application. (iii) shows the CVHR considering (iv) in a band form. It will be understood that (v) is closer to the actual apnea or hypopnea condition of (i).

### Summary of Processing of Third Embodiment of Sleep Condition Detection Apparatus

In the second embodiment, the sleep condition of the test subject was diagnosed based on the beat interval data and autonomous nerve indicator data of the test subject during sleep. In the third embodiment, the sleep condition of the test subject is further diagnosed based on the body movement data.

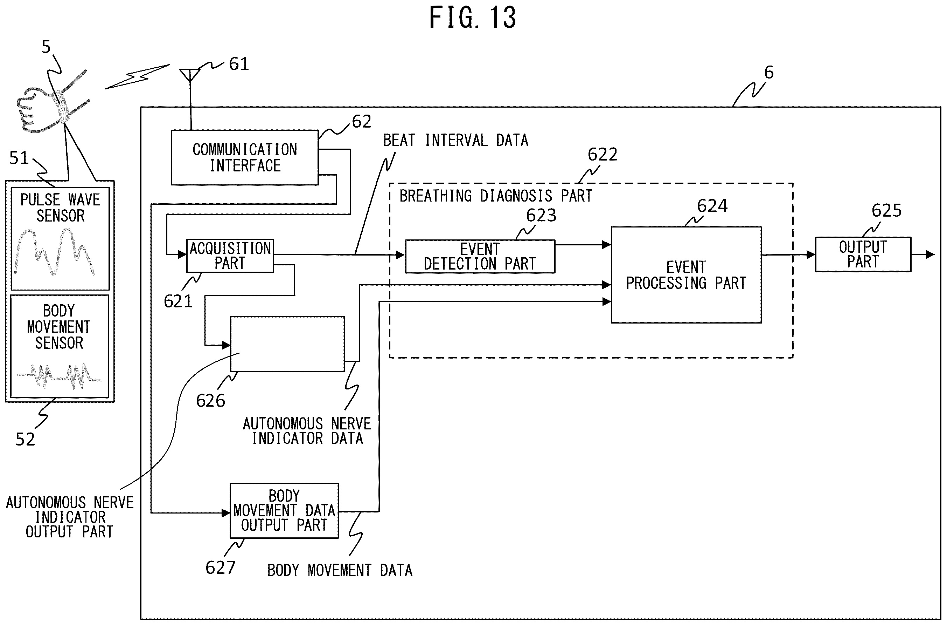

FIG. 13 is a view showing one example of the summary of processing of the third embodiment of a sleep condition detection apparatus according to the present disclosure.

At the wrist of the test subject, a wristband 5 having an optical type pulse wave sensor 51 for detecting the beating condition of the heart of the test subject, for example, the pulse, and a body movement sensor 52 is attached. The optical type pulse wave sensor 51, for example, has an optical type pulse wave sensor IC mounting an LED driver and a green color detection photodiode. It is possible to measure the intensity of reflected light at the time of firing the light of the LED toward the inside of the body by the photodiode to obtain a pulse wave signal. The pulse wave sensor may be not only an optical type, but also, for example, a pulse pressure detection type pulse wave sensor.

The body movement sensor 52 is, for example, a motion sensor having an acceleration sensor. The body movement sensor may also have a gyroscope. A plurality of acceleration sensors can also be used to detect motion vector quantities in three dimensions. Furthermore, it is possible to calculate the amount of movement of the wrist of the test subject.

The beat signal detected by the optical type pulse wave sensor 51 of the wristband 5 and the body movement signal detected by the body movement sensor 52 are sent by wireless communication, for example WiFi or Bluetooth^{®}, to the sleep condition detection apparatus 6.

The beat signal and the body movement signal sent from the wristband 5 are received by the communication interface 62 through the antenna 61 of the sleep condition detection apparatus 6.

An acquisition part 621 acquires beat interval data showing the interval of beats, for example, the time interval of the adjoining peak values of the pulse wave, from the beat signal showing the beating condition of the heart of the test subject. The beat interval data is input to a breathing diagnosis part 622. Alternatively, the beat interval data is input to an autonomous nerve indicator output part 626.

The autonomous nerve indicator output part 626 calculates the autonomous nerve indicator (LF/HF) data based on the beat interval data. The autonomous nerve indicator data output from the autonomous nerve indicator output part 626 is input to the breathing diagnosis part 622.

A body movement data output part 627 acquires the body movement signal of the test subject and outputs body movement data showing the amount of movement of the wrist of the test subject. If the test subject suffers from an apnea or hypopnea condition during sleep, it is known that when exceeding a limit and resuming breathing, along with body movement, a waking response is momentarily caused and breathing is resumed. By measuring the body movement during sleep, it is possible to judge the possibility of occurrence of apnea or hypopnea.

The breathing diagnosis part 622 uses the beat interval data, autonomous nerve indicator data, and body movement data to diagnose an apnea or hypopnea sleep condition of the test subj ect.

An event detection part 623 of the breathing diagnosis part 622 smooths the beat interval data. Furthermore, the event detection part 623 uses the conventional algorithm for detecting CVHR flags from a pulse wave shown in FIG. 3 to detect CVHR events. The detected CVHR event data is input to an event processing part 624.

An autonomous nerve indicator output part 726 outputs autonomous nerve indicator (LF/HF) data based on the beat interval. The autonomous nerve indicator data output from the autonomous nerve indicator output part 626 is input to the event processing part 624.

The event processing part 624 excludes from the detected CVHR event data the CVHR event data corresponding to the period where the beat interval becomes within a predetermined range.

The event processing part 624 further filters the processed CVHR event data showing apnea based on the autonomous nerve indicator data.

The event processing part 624 further filters the CVHR event data filtered on the basis of the autonomous nerve indicator data based on the body movement data. The filtering, for example, judges CVHR event data when the amount of body movement is a predetermined body movement indicator threshold or more as CVHR event data showing apnea. When the amount of body movement is large, it is diagnosed that the test subject has undergone a momentary waking response due to the apnea response and there is a high possibility of the test subject suffering from an apnea or hypopnea sleep condition.

Based on the results of detection by the event processing part 624, the output part 625 outputs CVHR event data as an apnea indicator. Instead of the CVHR event data or together with the CVHR event data, it is also possible to output an indicator of breathing per unit time of detection, for example, a value corresponding to the apnea hypopnea index (AHI) of the combined number of times stopped breathing and shallow breathing per hour.

The apnea indicator is input to a not shown display part built into the sleep condition detection apparatus 6. On the display screen, for example, the apnea hypopnea index is displayed.

The apnea indicator is input to a not shown display part built into the sleep condition detection apparatus 6. On the display screen, for example, a value corresponding to the apnea hypopnea index is displayed.

According to the sleep condition detection apparatus 6 of the present embodiment, it is possible to simply and conveniently further improve the precision of detection of an apnea or hypopnea condition at the time of sleep.

### Example of Constitution of Third Embodiment of Sleep Condition Detection Apparatus

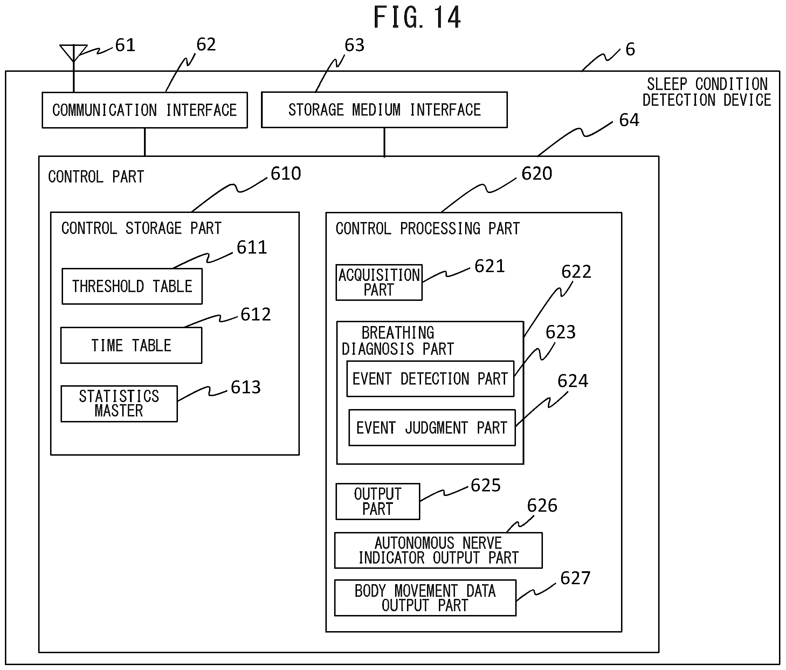

FIG. 14 is a view showing one example of the configuration of the third embodiment of a sleep condition detection apparatus.

The sleep condition detection apparatus 6 may also have a communication interface 62 communicating with the outside through the antenna 61 and a control part 64 connected to the communication interface 62. Furthermore, it may also have a storage medium interface 63 connected to an outside storage medium.

The control part 64 has a control storage part 610 and a control processing part 620. The control storage part 610 is comprised of one or more semiconductor memories.

Furthermore, the control storage part 610 may also temporarily store temporary data relating to predetermined processing. The control storage part 610 stores a threshold table 611 of the beat threshold, autonomous nerve indicator threshold, average range of beat interval, body movement threshold, etc., a time table 612 of the diagnosis unit time, autonomous nerve indicator output unit time, etc., statistics master 613 for statistical processing, etc.

The control processing part 620 has an acquisition part 621, breathing diagnosis part 622, event detection part 623, event processing part 624, output part 625, autonomous nerve indicator output part 626, body movement data output part 626, etc.

### Flow of Sleep Condition Detection Processing of Third Embodiment

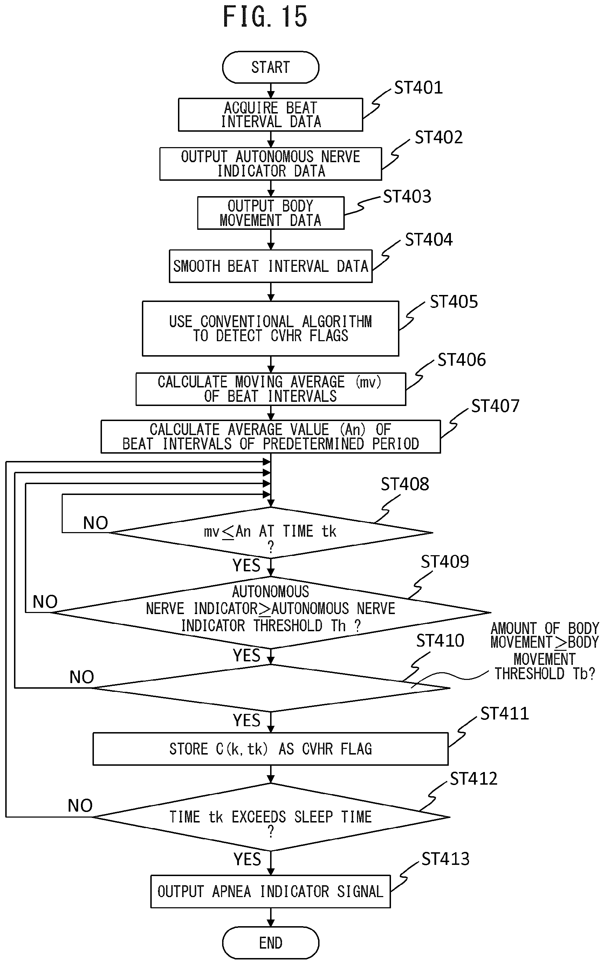

FIG. 15 is a view showing one example of sleep condition detection processing performed in the third embodiment.

The flow of sleep condition detection processing of this example is processing in which one time's worth of sleep time, for example, six hours' worth, of the beat signal and body movement signal of the test subject has already been sent from the wristband 5 to the sleep condition detection apparatus 6 and stored in the control storage part 610. The sleep condition detection processing can be performed all at once. Alternatively, for example, it is also possible to acquire the beat signal in one hour units and perform the diagnosis processing every one hour.

The acquisition part 621 uses the beat signal stored in the control storage part 610 to acquire beat interval data showing the beat interval (ST401).

The autonomous nerve indicator output part 626 outputs the autonomous nerve indicator data based on the beat interval data (ST402).

The body movement data output part 626 outputs the data based on the body movement signal stored in the control storage part 610 (ST403).

The event detection part 623 smooths the beat interval data (ST404). Furthermore, the event detection part 623 uses the conventional algorithm for detecting the CVHR flags from a pulse wave shown in FIG. 3 to detect CVHR events (ST405). The event processing part 624 calculates the moving average (mv) of the beat interval data (ST406). The event processing part 624 calculates the average value (An) in a predetermined period, for example, every hour or one sleep period (ST407).

The event processing part 624 judges if the moving average (mv) is the average value (An) or less at a time tk where a CVHR flag is detected (ST408). When the moving average (mv) is more than the average value (An) (ST408: NO), the event processing part 624 returns to ST408 where it compares the moving average (mv) and average value (An) of the CVHR flags at the next time. When the moving average (mv) is the average value (An) or less (ST408: YES), the event processing part 624 judges if the autonomous nerve indicator at the time tk is a predetermined autonomous nerve indicator threshold Th or more (ST409).

When the autonomous nerve indicator falls below the predetermined autonomous nerve indicator threshold Th (ST409: NO), the event processing part 624 returns to ST408. When the autonomous nerve indicator at the time tk is the predetermined autonomous nerve indicator threshold Th or more (ST409: YES), the event processing part 624 judges if the amount of body movement at the time tk is the body movement threshold Tb or more (ST410). When the amount of body movement is less than the body movement threshold Tb (ST410: NO), the event processing part 624 returns to ST408.When the amount of body movement is the body movement threshold Tb or more (ST410: YES), the event processing part 624 stores the CVHR flag c(k, tk) at the time tk as a CVHR flag (ST411).

The event processing part 624 judges if the time tk exceeds the sleep time (ST412). When the time tk does not exceed the sleep time (ST410: NO), the routine returns to ST408. When the time tk exceeds the sleep time (ST412: YES), the output part 625 outputs the apnea indicator based on the stored CVHR flags (ST413). The processing flow then ends.

### Summary of Fourth Embodiment of Sleep Condition Detection Apparatus

In the first, second, and third embodiments, the sleep condition detection apparatus diagnosed the sleep condition of the test subject by receiving a sensor signal from a wristband attached to the wrist of the test subject. In the fourth embodiment, for example a sleep condition detection apparatus of a smartwatch type in whose wristband a sleep condition detection apparatus is built in will be explained.

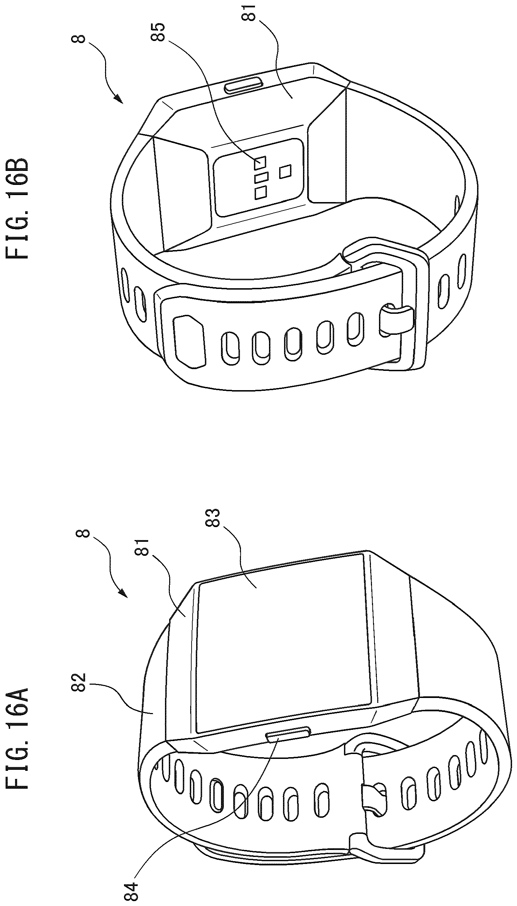

FIGS. 16A and 16B are perspective views of the outer shape showing one example of the fourth embodiment of a sleep condition detection apparatus. FIG. 16A is a perspective view of the outer shape seen from the front side, while FIG. 16B is a perspective view of the outer shape seen from the back side.

The sleep condition detection apparatus 8 has a main body part 81 and a wristband part 82. At the front side of the main body part 81, there is a display part 83, while at the side surface, there is an operating button 84. At the back side of the main body part 81, there is an optical type pulse wave sensor 85. A not shown acceleration sensor 86 is stored in the main body part 81.

### Example of Constitution of Fourth Embodiment of Sleep Condition Detection Apparatus

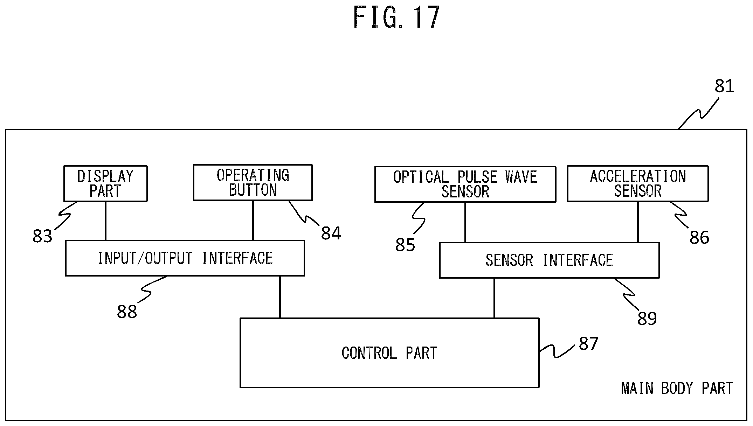

FIG. 17 is a view showing one example of the configuration of the main body part of the fourth embodiment of a sleep condition detection apparatus.

The main body part 81 has a display part 83, an operating button 84, optical type pulse wave sensor 85, acceleration sensor 86, control part 87, input/output interface 88, and sensor interface 88. The display part 83 and operating button 84 are connected to the control part 87 through the input/output interface 88, while the optical type pulse wave sensor 85 and acceleration sensor 86 are connected through the sensor interface 88 to the control part 87.

The beat signal from the optical type pulse wave sensor 85 and the body movement signal from the acceleration sensor 86 are input through the sensor interface 88 to the control part 87. The control part 87 uses the beat signal and body movement signal to diagnose the sleep condition of the test subject to whom the sleep condition detection apparatus 8 is attached. The apnea indicator output from the control part 87 is sent through the input/output interface 88, and the display part 83 displays the breathing indicator value. Alternatively, the operating button 84 controls the operation of the control part 87 through the input/output interface 88.

### Summary of Sleep Condition Display

The processing for display of the sleep condition of the test subject, for example, is processing which the sleep condition detection apparatus and the sleep display apparatus work together.

### Summary of Processing of Sleep Condition Detection Apparatus

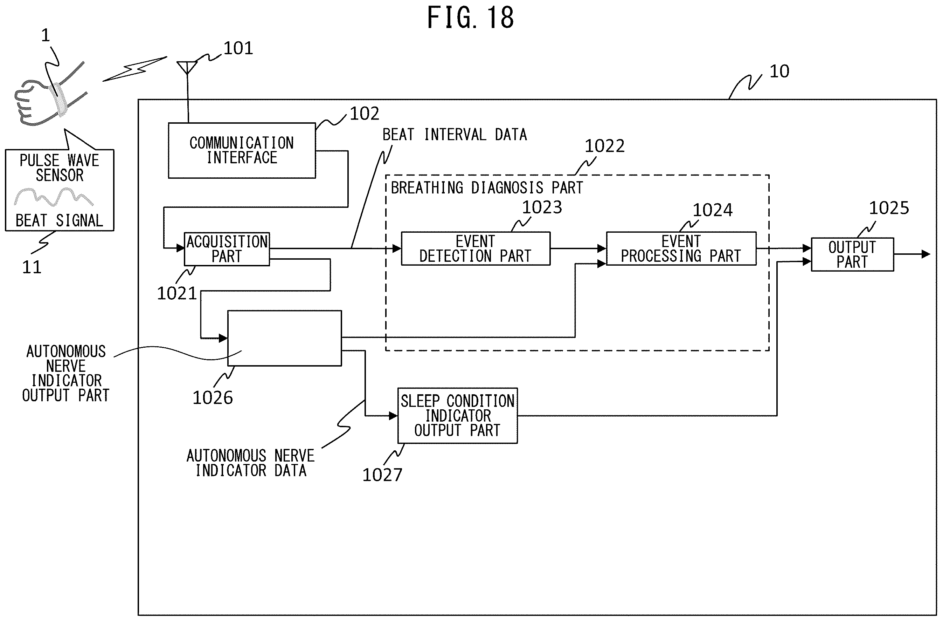

FIG. 18 is a view showing one example of the summary of processing of a sleep condition detection apparatus used in a sleep condition display.

The beat signal detected by the pulse wave sensor of the wristband 1 is sent by wireless communication, for example, WiFi or Bluetooth^{®}, to the sleep condition detection apparatus 10.

The beat signal sent from the wristband 1 is received by a communication interface 102 through an antenna 101 of the sleep condition detection apparatus 10.

An acquisition part 1021 acquires beat interval data showing the interval of beats from the beat signal showing the beating condition of the heart of the test subject, for example, the time interval of adjoining peak values of the pulse wave. The beat interval data is input to an breathing diagnosis part 422. Further, the beat interval data is output to an autonomous nerve indicator output part 426.

An event detection part 1023 of the breathing diagnosis part 1022 smooths the beat interval data. Furthermore, the event detection part 1023 uses the conventional algorithm for diagnosing CVHR flags from a pulse wave shown in FIG. 3 to detect CVHR events. The detected CVHR event data is input into an event processing part 1024.

An autonomous nerve indicator output part 1026 outputs autonomous nerve indicator (LF/HF) data based on the beat interval data. The autonomous nerve indicator data output from the autonomous nerve indicator output part 1026 is input to the event processing part 1024.

The event processing part 1024 excludes from the detected CVHR event data the CVHR event data corresponding to the period where the beat interval is within a predetermined range.

The event processing part 1024 further filters the processed CVHR event data showing apnea based on the autonomous nerve indicator data.

The sleep condition indicator output part 1026 uses the autonomous nerve indicator (LF/HF) data output from the autonomous nerve indicator output part 1025 to estimate a condition called a "sleep stage" showing REM sleep, nonREM sleep, or another sleep condition. It is known that at the time of nonREM sleep, the parasympathetic nerves are dominant. Therefore, the value of LF/HF is low and the value becomes lower as the sleep becomes deeper. On the other hand, it is known that at the time of REM sleep, the sympathetic nerves are dominant compared with nonREM sleep and the autonomous nerve condition becomes unstable, so if the value of LF/HF is high and the variation in the LF/HF of the predetermined range of time is large, REM sleep is judged. The sleep stage data output from the sleep condition indicator output part 1026 is input as sleep condition indicator data to the output part 1025.

Based on the results of detection by the event processing part 1024, the output part 1025 outputs CVHR event data as the apnea indicator. Instead of the CVHR event data or together with the CVHR event data, it is also possible to output an indicator of breathing per unit time of detection, for example, a value corresponding to the apnea hypopnea index (AHI) of the combined number of times of stopped breathing and shallow breathing per hour. Alternatively, the output part 1025 outputs sleep condition indicator data detecting a condition called the sleep stage showing REM sleep, nonREM sleep, or other sleep conditions.

As the apnea indicator, the CVHR event data or sleep condition indicator data is input to a not shown display part built in the sleep condition detection apparatus 10, and a value corresponding to for example the AHI is displayed on the display screen.

According to the sleep condition detection apparatus 10 of the present embodiment, it is possible to simply and conveniently improve the precision of detection of an apnea or hypopnea condition at the time of sleep.

### Example of Constitution of Sleep Condition Detection Apparatus

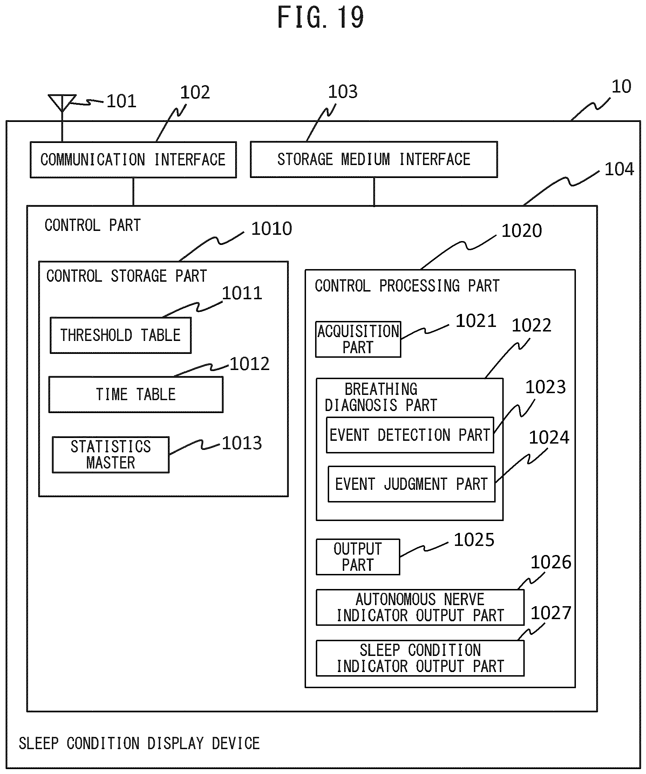

FIG. 19 is a view showing one example of the configuration of a sleep condition detection apparatus.

The sleep condition detection apparatus 10 may also have a communication interface 102 for communicating with the outside through an antenna 101 and a control part 104 connecting with the communication interface 102. Furthermore, it is also possible to have a storage medium interface 103 connecting with an outside storage medium.

The control part 104 has a control storage part 1010 and a control processing part 1020.

The control storage part 1010 may also temporarily store temporary data relating to predetermined processing. The control storage part 1010 stores a threshold table 1011 of the beat threshold, average range of beat interval, autonomous nerve indicator threshold, body movement threshold, etc., a time table 1012 of the diagnosis time, judgment time, autonomous nerve indicator calculation time, etc., a statistics master 1013 for statistical processing, etc.

The control processing part 1020 has an acquisition part 1021, breathing diagnosis part 1022, event detection part 1023, event processing part 10210, output part 1025, autonomous nerve indicator output part 1026, sleep condition indicator output part 1027, etc.

### Summary of Sleep Condition Display Screen

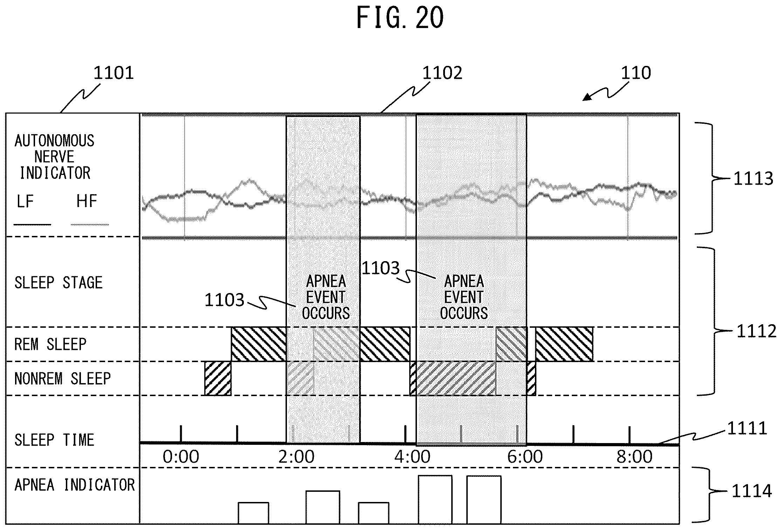

FIG. 20 is a view showing one example of a sleep display screen. The display screen is displayed on a display apparatus, for example, a liquid crystal display (LCD) or cathode ray tube device.

At the left side region of the display screen 110, a heading part 1101 is displayed. At the right side region of the heading part 1101, an indicator part 1102 is displayed. At the right side region of the display screen 110, a heading part 1101 may be displayed and at the left side region of the heading part 1101, an indicator part 1102 may be displayed.

At the indicator part 1102, a time axis 1111 showing the sleep passage time is displayed. At the time axis 1111, gradations or hour values are attached. At the heading part 1101, at the left side of the time axis 1111 displayed at the indicator part 1102, the heading "sleep time" may be displayed. Alternatively, instead of the time axis 1111, a time axis showing the time may be displayed. At the heading part 1101, the heading "time" may also be displayed corresponding to the time axis displayed at the indicator part 1102.

At the heading part 1101, the names of the indicators displayed at the indicator part 1102 are vertically displayed. As the names of the indicators, abbreviated names or, for example, the letters LF/HF or AHI are displayed. Furthermore, several indicator names may be omitted or changed. At the indicator part 1102, the sleep condition indicator 1112, autonomous nerve indicator 1113, and apnea indicator 1114 are displayed at predetermined common time intervals in time series so as to be positioned at the right side of the indicator names displayed at the heading part 1101. Alternatively, for example, the sleep condition indicator 1112 may be displayed as the "sleep stage" showing the condition called the sleep stage showing REM sleep, nonREM sleep, or other sleep condition.

When an indicator is further represented by a plurality of indicators, for example, when the sleep condition indicator is represented by the REM sleep condition and nonREM sleep condition, the heading part 1101 may, for example, display the names "REM sleep" and "nonREM sleep" as subheadings. At the indicator part 1102, at the right side of the respective subheadings, the REM sleep condition and nonREM sleep condition are displayed at predetermined common time intervals in time series.

As the representations of indicators displayed at predetermined common time intervals in time series, the sleep condition indicator 1112 is represented by, for example, a band graph as will be understood by the switching of the REM sleep condition and nonREM sleep condition. The autonomous nerve indicator 1113 with the relatively short variation time is, for example, displayed by a broken line graph of LF and HF. As the apnea indicator 1114, for example, when showing the apnea hypopnea index (AHI), it is expressed by a bar graph. The apnea hypopnea index is the combined number of times of stopped breathing and shallow breathing per hour. The representation of indicators displayed at predetermined common time intervals in time series are not limited to these. Various representations, for example, color displays, are also possible.

Furthermore, when a predetermined number of times of CVHR events occur in a predetermined time, for example, one hour, showing when the test subject is suffering from an apnea condition, they are displayed as a band region 113 of consecutive CVHR events within one hour on the display screen 110 overlapped on other indicators. This one hour is just one example. The time may be 10 minutes, 30 minutes, etc. A CVHR event is an event showing the occurrence of an apnea or hypopnea condition of the test subject.

The band region 113 of consecutive CVHR events is shown colored out or semitransparent. When semitransparent, the sleep condition indicator 1112, autonomous nerve indicator 1113, and apnea indicator 1114 can be visually confirmed. Alternatively, the band region 113 of consecutive CVHR events may be displayed on the display screen 110 overlapped on at least one of the indicator parts. Furthermore, the occurrence of an CVHR event, that is, an apnea/hypopnea event, may be explained in the display. Alternatively, CVHR events may be directly displayed overlapped.

### Example of Constitution of Sleep Condition Display Apparatus

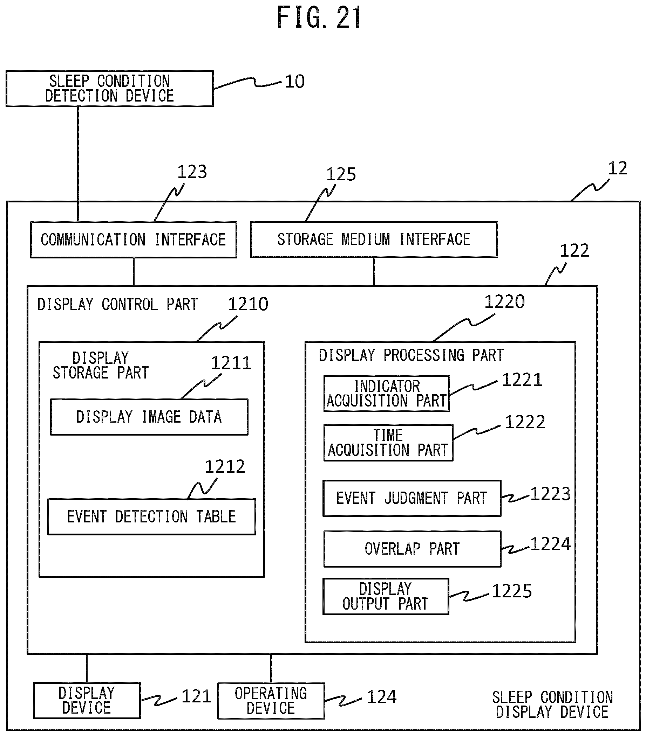

FIG. 21 is a view showing one example of the configuration of a sleep condition display apparatus.

The sleep condition display apparatus 12 has a display apparatus 121, display control part 122 connected to the display apparatus, communication interface 123 connected to the display control part 122 and communicating with an outside device, and operation device 124 connected to the display control part 122 and operating the apparatus. Furthermore, it may also have a storage medium interface 125 connected to the display control part 122 and connected with an outside storage medium. For example, the display apparatus 121 and the operation device 124 may also be provided integrally as a touch panel display.

For example, the sleep condition display apparatus 12 receives a sleep condition indicator signal, autonomous nerve indicator signal, and apnea indicator signal from the sleep condition detection apparatus 3 through the communication interface 123. Alternatively, the sleep condition display apparatus 12 can read sleep condition indicator data, autonomous nerve indicator data, and apnea indicator data stored in an outside storage medium through the storage medium interface 125.

The display control part 122 has a display storage part 1210 and a display processing part 1220. The display storage part 1210 is comprised of one or more semiconductor memories. For example, it has at least one of a RAM, flash memory, EPROM, EEPROM, or other nonvolatile memory. The display storage part 1210 stores a driver program, operating system program, application programs, data, etc. used for processing by the display processing part 1220.

For example, the display storage part 1210 stores as a driver program a device driver program for controlling the display apparatus 121 and the operation device 124. The computer program, for example, may also be installed from a CD-ROM, DVD-ROM, or other computer readable portable storage medium into the display storage part 1210 using a known setup program etc. Further, it may also be installed by being downloaded from a program server etc.

Furthermore, the display storage part 1210 may also temporarily store temporary data relating to predetermined processing. The display storage part 1210 stores the display elements, display patterns, and other display image data 1211 to be displayed on the display apparatus 121. Further, the display storage part 1210 for example stores an event detection table 1212 storing apnea indicator thresholds and other indicator thresholds related to the sleep time for detecting occurrence of CVHR events.

The display processing part 1220 has one or more processors and their peripheral circuits. The display processing part 1220 comprehensively controls the overall operation of the sleep condition display apparatus 12 and, for example, is an MCU.

The display processing part 1220 performs processing based on a program stored in the display storage part 1210 (operating system program, driver program, application programs, etc.) Further, the display processing part 1220 may also run a plurality of programs (application programs etc.) in parallel. The display processing part 1220 has an indicator acquisition part 1221, time point acquisition part 1222, event judgment part 1223, overlap part 1224, display output part 1225, etc.

These parts of the display processing part 1220 may also be mounted at the display control part 122 as independent integrated circuits, circuit modules, microprocessors, or firmware.

### Flow of Sleep Condition Display Processing

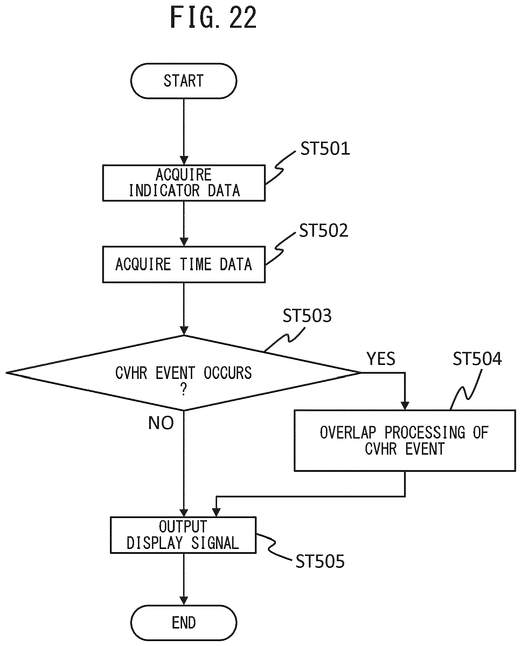

FIG. 22 is a view showing one example of a flow chart of sleep condition display processing.

The indicator acquisition part 1221 receives sleep condition indicator data, autonomous nerve indicator data, apnea indicator data, and CVHR event data of the test subject through the communication interface 123 from the sleep condition detection apparatus 3 (ST501). The time point acquisition part 1222 acquires the sleep start time and sleep end time of the test subject through the operation device 124 and calculates the sleep time of the test subject (ST502). The event judgment part 1223, for example, judges if a predetermined number of times of CVHR events occur in a predetermined time showing when the test subject suffers from an apnea condition, for example, 1 hour (ST503). When a predetermined number of times of CVHR events occur in a predetermined time (ST503: YES), the overlap part 1224 displays the CVHR events in a predetermined time overlapped on other indicators as a continuous band region 73 on the display screen 7. It performs CVHR event overlap display processing (ST504). The display output part 1225 outputs a display signal to the display apparatus 121. When a predetermined number of times of CVHR events do not occur in a predetermined time (ST503: NO), the display output part 1225 outputs a display signal to the display apparatus 121. If the display output part 1225 outputs a display signal, it ends the flow of processing.

In the present embodiment, the sleep condition detection apparatus 3 and the sleep condition display apparatus 12 were explained as separate apparatuses, but the sleep condition detection apparatus and the sleep condition display apparatus may also be a single apparatus.

When the sleep condition detection apparatus and the sleep condition display apparatus are made an integral unit, a single processing part may perform the sleep condition detection processing and the sleep condition display processing.

In the above explanation, the pulse was explained as an example of the beating condition of the heart of the test subject, but the heartbeat may also be used as the beating condition of the heart of the test subject.

The sleep condition detection apparatus according to the present invention may also be made an embodiment by linkup between the server apparatus and the client apparatus.

A person skilled in the art would understand that various changes, substitutions, and corrections may be made without departing from the spirit and scope of the present invention.

## Claims

1. A sleep condition detection apparatus comprising
an acquisition part for acquiring beat interval data of a test subject in a predetermined period including a sleep period,
an event detection part for detecting cyclic variation of heart rate (CVHR) event data from the beat interval data,
an event processing part for performing exclusion processing for excluding from the CVHR event data the CVHR event data of a period where the beat interval becomes within a predetermined range, and
an output part for outputting an apnea indicator signal related to the processed CVHR event data.

2. The sleep condition detection apparatus according to claim 1, further comprising an autonomous nerve indicator output part for outputting autonomous nerve indicator data showing an autonomous nerve indicator based on the beat interval data,
the event processing part further excluding from the processed CVHR event data the CVHR event data of a period where the autonomous nerve indicator data falls below a predetermined threshold.

3. The sleep condition detection apparatus according to claim 1 further comprising
an autonomous nerve indicator output part for outputting autonomous nerve indicator data showing an autonomous nerve indicator based on the beat interval data and
a breathing diagnosis part for diagnosing an apnea or hypopnea sleep condition of the test subject based on the processed CVHR event data and the autonomous nerve indicator data,
the output part outputting an apnea indicator signal showing the results of diagnosis.

4. The sleep condition detection apparatus according to claim 3, further comprising a body movement data output part for acquiring a body movement signal of the test subject and outputting body movement data,
the breathing diagnosis part diagnosing an apnea or hypopnea sleep condition of the test subject based on the beat interval data, the autonomous nerve indicator data, and the body movement data.

5. The sleep condition detection apparatus according to claim 4, wherein the breathing diagnosis part further comprises
an event detection part for detecting CVHR event data from the beat interval data and
an event processing part for excluding from the CVHR event data the CVHR event data of a period where the beat interval becomes within a predetermined range, excludes from the CVHR event data the CVHR event data of a period where the autonomous nerve indicator data becomes less than a predetermined autonomous nerve indicator threshold, and excludes the CVHR event data of a period where the body movement data becomes less than a predetermined body movement threshold.

6. The sleep condition detection apparatus according to claim 3 or 4, wherein the breathing diagnosis part diagnoses an apnea or hypopnea sleep condition of the test subject based on data of a plurality of test subjects.

7. A sleep condition detection method comprising
acquiring beat interval data of a test subject in a predetermined period including a sleep period,
detecting cyclic variation of heart rate (CVHR) event data from the beat interval data,
performing exclusion processing excluding from the CVHR event data the CVHR event data of a period where the beat interval becomes within a predetermined range, and
outputting an apnea indicator signal relating to the processed CVHR event data.

8. The sleep condition detection method according to claim 7, further comprising
outputting autonomous nerve indicator data showing an autonomous nerve indicator from the beat interval data,
diagnosing an apnea or hypopnea sleep condition of the test subject based on the processed CVHR event data and the autonomous nerve indicator data, and
outputting an apnea indicator signal showing the diagnosis results.

9. The sleep condition detection method according to claim 7, further comprising
acquiring a sleep condition indicator based on beat interval data of the test subject,
acquiring an autonomous nerve indicator based on the beat interval data,
acquiring CVHR events showing the occurrence of an apnea condition of the test subject based on the sleep condition indicator and the autonomous nerve indicator,
acquiring an apnea indicator based on the CVHR events,
displaying the sleep condition indicator, the autonomous nerve indicator, and the apnea indicator at predetermined common time intervals in a time series, and
displaying the CVHR events overlapped with other indicators.

10. The sleep condition detection method according to claim 9, wherein the CVHR events are displayed overlapped with the sleep condition indicator, the autonomous nerve indicator, and the apnea indicator so as to cover over the display of the indicators.

11. The sleep condition detection method according to claim 9, wherein the CVHR events are displayed overlapped with the sleep condition indicator, the autonomous nerve indicator, and the apnea indicator so as to enable the display of the indicators to be viewed.

12. A sleep condition detection program for running a computer,
which program makes a computer run a sleep condition detection method according to claims 7 to 11.
